# EUROPEAN PATENT SPECIFICATION

(11) **EP 2 710 150 B1**
(45) Date of publication and mention of the grant of the patent: **24.08.2016**
(21) Application number: 12785674.8
(22) Date of filing: 15.05.2012
(51) Int. Cl.: C12Q 1/68

(54) **IDENTIFICATION OF THE CAUSATIVE MUTATION FOR INHERITED CONNECTIVE TISSUE DISORDERS IN EQUINES**
IDENTIFIKATION KAUSATIVER MUTATIONEN FÜR VERERBTE BINDEGEWEBSERKRANKUNGEN BEI PFERDEN
IDENTIFICATION DE LA MUTATION RESPONSABLE DE TROUBLES DU TISSU CONJONCTIF HÉRÉDITAIRES CHEZ LES ÉQUIDÉS

(30) Priority: 16.05.2011 US 201161486464 P
(43) Date of publication of application: 26.03.2014
(73) Proprietor: Cornell University, Ithaca, NY 14850 (US)
(72) Inventor: WINAND, Nena, J., Groton, NY 13073 (US)
(74) Representative: O'Connell, Maura
(86) International application number: PCT/US2012/037981
(87) International publication number: WO 2012/158711

(56) References cited:
- WO-A1-2008/121727
- US-A1- 2004 137 470
- US-A1- 2009 258 360
- US-A1- 2011 265 193
- VL MARSHALL ET AL: "Cutaneous asthenia in a Warmblood foal", AUSTRALIAN VETERINARY JOURNAL, vol. 89, no. 3, 16 February 2011 (2011-02-16), pages 77-81, XP055147733, ISSN: 0005-0423, DOI: 10.1111/j.1751-0813.2010.00683.x
- HEATHER N. YEOWELL ET AL: "Mutations in the Lysyl Hydroxylase 1 Gene That Result in Enzyme Deficiency and the Clinical Phenotype of Ehlers-Danlos Syndrome Type VI", MOLECULAR GENETICS AND METABOLISM, vol. 71, no. 1-2, 1 September 2000 (2000-09-01), pages 212-224, XP055147978, ISSN: 1096-7192, DOI: 10.1006/mgme.2000.3076
- Anonymous: "Warmblood Fragile Foal Syndrome (Ehlers-Danlos Syndrom)", VET-Magazin.de, 1 December 2012 (2012-12-01), XP002731439, Retrieved from the Internet: URL:http://dfpcorec-p.internal.epo.org/wf/ web/citenpl/citenpl.html?_url=http%3A%2F%2 Fwww.vet-magazin.de%2Fprint%2FLYmpheqFg-mK o-O-7jXSp1.pdf [retrieved on 2014-10-21]
- DATABASE GENBANK [Online] 30 December 2010 'Equine Articular Cartilage cDNA Library Equus caballus cDNA clone CT020012B10E10', XP003030299 Database accession no. CX595913 - CT020012B10E10
- DATABASE GENBANK [Online] 30 December 2010 'Equine Articular Cartilage cDNA Library Equus caballus cDNA clone CT020018B10E01', XP003030300 Database accession no. CX597645 - CT020018B10E01

### SEQUENCE LISTING

<110> Cornell University
<120> IDENTIFICATION OF THE CAUSATIVE MUTATION FOR INHERITED CONNECTIVE TISSUE DISORDERS IN EQUINES AND METHODS FOR TESTING FOR SAME
<130> 018617.00394
<150> 61/486,464
   <151> 2011-05-16
<160> 28
<170> PatentIn version 3.5
<210> 1
   <211> 350
   <212> DNA
   <213> horse
<400> 1
   atagctgtca ctccacaagg cacaagggct gcttggctgg ggtggctcag atgggagaat        60
   ggggggtact aggggaaggg cccagcttcc tcttggggaa actgacgctt cctgttggga         120
   aactgacact tcctgtctcc cagggcaggg gctgtcggtt cctgcgttac aactgctcca        180
   tccgagcccc acggaagggc tggaccctca tgcaccccgg acgactcacg cactaccacg        240
   agggactccc caccaccaag ggcacccgct acatcgcggt ctccttcgtc gatccctaat        300
   cggccaagcc tggccacttc ggaccttttc ctcgttgcca acaaccactg                   350
<210> 2
   <211> 2992
   <212> DNA
   <213> horse
<220>
   <221> misc_feature
   <222> (2374)..(2374)
   <223> n is a, c, g, or t
<220>
   <221> misc_feature
   <222> (2634)..(2634)
   <223> n is a, c, g, or t
<400> 2
   agtttccagc ctgccagcgc ctgctggctg cccggacgtc cccagacctc ggccatgcgg        60
   cctctgctgc tcctggcccc gctgggctgg ctgctcctgg ccgaagcgaa gggcgacgcc       120
   aagccggagg acaacctctt agtcctcacg gtggccacga aggagtccga ggggttccga       180
   cgcttcaagc gctcagccca gttcttcaac tacaagatcc aggcgctggg cctgggggag       240
   gactgggacg gggacaagga gacgtcagcg ggcggcgggc tgaaggttcg gctgctgaag       300
   aaagctctgg agaagcatgc agacaaagag aacctggtca ttctcttcac agacagctat       360
   gatgtggtat ttgcctcggg gccccgagag ctcctgaaga agttccggca ggccaggagc 420
   caggtggtct tctcggccga ggagctcatc taccccgacc gcaggctgga ggccaagtac 480
   ccggtggtgt ccgatggcaa gaggttcctg ggctctggag gcttcatcgg ttatgccccc 540
   aacctcagca aactggtggc tgagtgggag ggccaggaca gcgacagtga ccagctgttt 600
   tataccaaga tcttcttgga cccagagaag agggagcgga tcaacatcac cctggaccac 660
   cgctgccgta tcttccagaa cctggttgga gccttagatg aggtcgtgct caagtttgaa 720
   atgggccatg tgagggcgcg gaacctggcc tacgacaccc tccccgtcct gattcatggc 780
   aacgggccca ccaagctgca gctgaactac ctgggcaact atatccctcg cttctggacc 840
   ttcgagacgg gctgcacggt gtgtgacgag ggcctgcgca gcctcaaggg cattggggat 900
   gaagctctgc ctgtggtctt ggtcggcgtg ttcatcgagc agcccacgcc gttcctgtcc 960
   ctgttcttcc agcggcttct gcgcctgcat tacccccgga aacagctgcg gctttttatt 1020
   cataaccatg agcagcacca caaggctcag gtggagcagt tcctggcaga gcatggcggc 1080
   gagtacaagt ctgtgaaact ggtgggcccc gaggtgcggg tggcaaacgc cgatgccagg 1140
   aacatgggcg cggacctgtg ccggcaggac cgtggctgca cctactactt cagtgtggat 1200
   gccgacgtgg ccctgaccga gcccaagacc ctgcgactgc tgattgagca gaacaagaat 1260
   gtcatcgccc cgttgatgac ccgccacggg aggctgtggt cgaacttctg gggggcaatg 1320
   agtgcagatg gctactacgc ccgctccgag gactacgtgg acattgtgca ggggcggcgt 1380
   gttggcgtct ggaacgtgcc ctacatctcg aacatttacc tgatcaaggg cagtgccctg 1440
   cgggctgagc tgcagcagac agatctgttc caccacagca agctggatgc cgacatggcc 1500
   ttctgtgcca atatccggca gcaggatgtg ttcatgttcc tgaccaaccg gcacaccttc 1560
   ggccacctgc tctccctgga cagctaccag accacccacc tccacaacga cctctgggag 1620
   gtgtttagca accccgagga ctggaaggag aagtacatcc atgagaacta caccagggcc 1680
   ctggcgggga agctggtgga gatgccttgc ccggatgtct actggttccc catcttcacg 1740
   gagacggcct gtgacgagct ggtggaggag atggagcact acggccagtg gtctctggga 1800
   gacaataagg acaaccgcat ccagggtggc tacgaaaatg tgccgaccat cgacatccac 1860
   atgaaccaga tcagctttga gcgggagtgg cacaagttcc tggtggagta catcgccccc 1920
   atgacagaga agctgtatcc aggctactac accagggccc agttcgacct ggcctttgtt 1980
   gtccgctaca agcctgacga gcagccctcg ctgatgcccc accacgatgc ctccaccttc 2040
   actgtcaaca tcgccctgaa ccgggtcggg gtggattacg agggcggggg ctgtcggttc 2100
   ctgcgttaca actgctccat ccgagcccca cggaagggct ggaccctcat gcaccccgga 2160
   cgactcacgc actaccacga gggactcccc accaccaagg gcacccgcta catcgcggtc 2220
   tccttcgtcg atccctaatc ggccaagcct ggccacttcg gaccttttcc tcgttgccaa    2280
   caaccactgc ccagcagcct ctggggcctt ggggtcccag ggaacctggt ccagcctccg    2340
   ggctcttgac ctcccattgc tttcggagcc gccntcggag agactgggcc gcaggccaga    2400
   ggcagagcac acctccttgg ctggggcttt cctggtgttc tgctccccac cccgggagat    2460
   ggggtccacg ctcactgcct tgtaacagct catcctctcc cacctgttct cctgaaaagc    2520
   ccggtccctc ttcctctgcc tcttccatgg gcccagacct gagcagaacc gggcttaccc    2580
   agctgcccag agagactcta ggggccagaa gccatgcccc agagctccca ggcngggctg    2640
   ccacccggga acttctgctt caagcttcag ggtagacaca gagacctgga tgagactcaa     2700
   gtcccctccc tgatcctggg cctgctgaag ccccttcctc catggctcct gtcatgagag     2760
   caaaacattg tcgcctggag acggtgactc ggaaagcctc ctgggagaca ggaaaggcat    2820
   cgatgccaca gctccatcct ctacttgacc cttgctggcg ggaggggagt gatatgtcca    2880
   cacactgcac tgcgtcatcc tgttccggat gcctccggag agagggacgg acagtcagaa    2940
   acaagggagt ttctattaaa ggccatccaa accaaaaaaa aaaaaaaaaa aa            2992
<210> 3
   <211> 727
   <212> PRT
   <213> horse
<400> 3
   Met Arg Pro Leu Leu Leu Leu Ala Pro Leu Gly Trp Leu Leu Leu Ala
   1               5                   10                  15
   Glu Ala Lys Gly Asp Ala Lys Pro Glu Asp Asn Leu Leu Val Leu Thr
               20                  25                  30
   Val Ala Thr Lys Glu Ser Glu Gly Phe Arg Arg Phe Lys Arg Ser Ala
           35                  40                  45
   Gln Phe Phe Asn Tyr Lys Ile Gln Ala Leu Gly Leu Gly Glu Asp Trp
       50                  55                  60
   Asp Gly Asp Lys Glu Thr Ser Ala Gly Gly Gly Leu Lys Val Arg Leu
   65                  70                  75                  80
   Leu Lys Lys Ala Leu Glu Lys His Ala Asp Lys Glu Asn Leu Val Ile
                   85                  90                  95
   Leu Phe Thr Asp Ser Tyr Asp Val Val Phe Ala Ser Gly Pro Arg Glu
               100                 105                 110
   Leu Leu Lys Lys Phe Arg Gln Ala Arg Ser Gln Val Val Phe Ser Ala
           115                 120                 125
   Glu Glu Leu Ile Tyr Pro Asp Arg Arg Leu Glu Ala Lys Tyr Pro Val
       130                 135                 140
   Val Ser Asp Gly Lys Arg Phe Leu Gly Ser Gly Gly Phe Ile Gly Tyr
   145                 150                 155                 160
   Ala Pro Asn Leu Ser Lys Leu Val Ala Glu Trp Glu Gly Gln Asp Ser
                   165                 170                 175
   Asp Ser Asp Gln Leu Phe Tyr Thr Lys Ile Phe Leu Asp Pro Glu Lys
               180                 185                 190
   Arg Glu Arg Ile Asn Ile Thr Leu Asp His Arg Cys Arg Ile Phe Gln
           195                 200                 205
   Asn Leu Val Gly Ala Leu Asp Glu Val Val Leu Lys Phe Glu Met Gly
       210                 215                 220
   His Val Arg Ala Arg Asn Leu Ala Tyr Asp Thr Leu Pro Val Leu Ile
   225                 230                 235                 240
   His Gly Asn Gly Pro Thr Lys Leu Gln Leu Asn Tyr Leu Gly Asn Tyr
                   245                 250                 255
   Ile Pro Arg Phe Trp Thr Phe Glu Thr Gly Cys Thr Val Cys Asp Glu
               260                 265                 270
   Gly Leu Arg Ser Leu Lys Gly Ile Gly Asp Glu Ala Leu Pro Val Val
           275                 280                 285
   Leu Val Gly Val Phe Ile Glu Gln Pro Thr Pro Phe Leu Ser Leu Phe
       290                 295                 300
   Phe Gln Arg Leu Leu Arg Leu His Tyr Pro Arg Lys Gln Leu Arg Leu
   305                 310                 315                 320
   Phe Ile His Asn His Glu Gln His His Lys Ala Gln Val Glu Gln Phe
                   325                 330                 335
   Leu Ala Glu His Gly Gly Glu Tyr Lys Ser Val Lys Leu Val Gly Pro
               340                 345                 350
   Glu Val Arg Val Ala Asn Ala Asp Ala Arg Asn Met Gly Ala Asp Leu
           355                 360                 365
   Cys Arg Gln Asp Arg Gly Cys Thr Tyr Tyr Phe Ser Val Asp Ala Asp
       370                 375                 380
   Val Ala Leu Thr Glu Pro Lys Thr Leu Arg Leu Leu Ile Glu Gln Asn
   385                 390                 395                 400
   Lys Asn Val Ile Ala Pro Leu Met Thr Arg His Gly Arg Leu Trp Ser
                   405                 410                 415
   Asn Phe Trp Gly Ala Met Ser Ala Asp Gly Tyr Tyr Ala Arg Ser Glu
               420                 425                 430
   Asp Tyr Val Asp Ile Val Gln Gly Arg Arg Val Gly Val Trp Asn Val
           435                 440                 445
   Pro Tyr Ile Ser Asn Ile Tyr Leu Ile Lys Gly Ser Ala Leu Arg Ala
       450                 455                 460
   Glu Leu Gln Gln Thr Asp Leu Phe His His Ser Lys Leu Asp Ala Asp
   465                 470                 475                 480
   Met Ala Phe Cys Ala Asn Ile Arg Gln Gln Asp Val Phe Met Phe Leu
                   485                 490                 495
   Thr Asn Arg His Thr Phe Gly His Leu Leu Ser Leu Asp Ser Tyr Gln
               500                 505                 510
   Thr Thr His Leu His Asn Asp Leu Trp Glu Val Phe Ser Asn Pro Glu
           515                 520                 525
   Asp Trp Lys Glu Lys Tyr Ile His Glu Asn Tyr Thr Arg Ala Leu Ala
       530                 535                 540
   Gly Lys Leu Val Glu Met Pro Cys Pro Asp Val Tyr Trp Phe Pro Ile
   545                 550                 555                 560
   Phe Thr Glu Thr Ala Cys Asp Glu Leu Val Glu Glu Met Glu His Tyr
                   565                 570                 575
   Gly Gln Trp Ser Leu Gly Asp Asn Lys Asp Asn Arg Ile Gln Gly Gly
               580                 585                 590
   Tyr Glu Asn Val Pro Thr Ile Asp Ile His Met Asn Gln Ile Ser Phe
           595                 600                 605
   Glu Arg Glu Trp His Lys Phe Leu Val Glu Tyr Ile Ala Pro Met Thr
       610                 615                 620
   Glu Lys Leu Tyr Pro Gly Tyr Tyr Thr Arg Ala Gln Phe Asp Leu Ala
   625                 630                 635                 640
   Phe Val Val Arg Tyr Lys Pro Asp Glu Gln Pro Ser Leu Met Pro His
                   645                 650                 655
   His Asp Ala Ser Thr Phe Thr Val Asn Ile Ala Leu Asn Arg Val Gly
               660                 665                 670
   Val Asp Tyr Glu Gly Gly Gly Cys Arg Phe Leu Arg Tyr Asn Cys Ser
           675                 680                 685
   Ile Arg Ala Pro Arg Lys Gly Trp Thr Leu Met His Pro Gly Arg Leu
       690                 695                 700
   Thr His Tyr His Glu Gly Leu Pro Thr Thr Lys Gly Thr Arg Tyr Ile
   705                 710                 715                 720
   Ala Val Ser Phe Val Asp Pro
                   725
<210> 4
   <211> 2992
   <212> DNA
   <213> horse
<400> 4
   agtttccagc ctgccagcgc ctgctggctg cccggacgtc cccagacctc ggccatgcgg      60
   cctctgctgc tcctggcccc gctgggctgg ctgctcctgg ccgaagcgaa gggcgacgcc     120
   aagccggagg acaacctctt agtcctcacg gtggccacga aggagtccga ggggttccga     180
   cgcttcaagc gctcagccca gttcttcaac tacaagatcc aggcgctggg cctgggggag     240
   gactgggacg gggacaagga gacgtcagcg ggcggcgggc tgaaggttcg gctgctgaag     300
   aaagctctgg agaagcatgc agacaaagag aacctggtca ttctcttcac agacagctat     360
   gatgtggtat ttgcctcggg gccccgagag ctcctgaaga agttccggca ggccaggagc     420
   caggtggtct tctcggccga ggagctcatc taccccgacc gcaggctgga ggccaagtac     480
   ccggtggtgt ccgatggcaa gaggttcctg ggctctggag gcttcatcgg ttatgccccc     540
   aacctcagca aactggtggc tgagtgggag ggccaggaca gcgacagtga ccagctgttt     600
   tataccaaga tcttcttgga cccagagaag agggagcgga tcaacatcac cctggaccac     660
   cgctgccgta tcttccagaa cctggttgga gccttagatg aggtcgtgct caagtttgaa     720
   atgggccatg tgagggcgcg gaacctggcc tacgacaccc tccccgtcct gattcatggc     780
   aacgggccca ccaagctgca gctgaactac ctgggcaact atatccctcg cttctggacc     840
   ttcgagacgg gctgcacggt gtgtgacgag ggcctgcgca gcctcaaggg cattggggat 900
   gaagctctgc ctgtggtctt ggtcggcgtg ttcatcgagc agcccacgcc gttcctgtcc 960
   ctgttcttcc agcggcttct gcgcctgcat tacccccgga aacagctgcg gctttttatt 1020
   cataaccatg agcagcacca caaggctcag gtggagcagt tcctggcaga gcatggcggc 1080
   gagtacaagt ctgtgaaact ggtgggcccc gaggtgcggg tggcaaacgc cgatgccagg 1140
   aacatgggcg cggacctgtg ccggcaggac cgtggctgca cctactactt cagtgtggat 1200
   gccgacgtgg ccctgaccga gcccaagacc ctgcgactgc tgattgagca gaacaagaat 1260
   gtcatcgccc cgttgatgac ccgccacggg aggctgtggt cgaacttctg gggggcaatg 1320
   agtgcagatg gctactacgc ccgctccgag gactacgtgg acattgtgca ggggcggcgt 1380
   gttggcgtct ggaacgtgcc ctacatctcg aacatttacc tgatcaaggg cagtgccctg 1440
   cgggctgagc tgcagcagac agatctgttc caccacagca agctggatgc cgacatggcc 1500
   ttctgtgcca atatccggca gcaggatgtg ttcatgttcc tgaccaaccg gcacaccttc 1560
   ggccacctgc tctccctgga cagctaccag accacccacc tccacaacga cctctgggag 1620
   gtgtttagca accccgagga ctggaaggag aagtacatcc atgagaacta caccagggcc 1680
   ctggcgggga agctggtgga gatgccttgc ccggatgtct actggttccc catcttcacg 1740
   gagacggcct gtgacgagct ggtggaggag atggagcact acggccagtg gtctctggga 1800
   gacaataagg acaaccgcat ccagggtggc tacgaaaatg tgccgaccat cgacatccac 1860
   atgaaccaga tcagctttga gcgggagtgg cacaagttcc tggtggagta catcgccccc 1920
   atgacagaga agctgtatcc aggctactac accagggccc agttcgacct ggcctttgtt 1980
   gtccgctaca agcctgacga gcagccctcg ctgatgcccc accacgatgc ctccaccttc 2040
   actgtcaaca tcgccctgaa ccgggtcggg gtggattacg agggcagggg ctgtcggttc 2100
   ctgcgttaca actgctccat ccgagcccca cggaagggct ggaccctcat gcaccccgga 2160
   cgactcacgc actaccacga gggactcccc accaccaagg gcacccgcta catcgcggtc 2220
   tccttcgtcg atccctaatc ggccaagcct ggccacttcg gaccttttcc tcgttgccaa 2280
   caaccactgc ccagcagcct ctggggcctt ggggtcccag ggaacctggt ccagcctccg 2340
   ggctcttgac ctcccattgc tttcggagcc gccatcggag agactgggcc gcaggccaga 2400
   ggcagagcac acctccttgg ctggggcttt cctggtgttc tgctccccac cccgggagat 2460
   ggggtccacg ctcactgcct tgtaacagct catcctctcc cacctgttct cctgaaaagc 2520
   ccggtccctc ttcctctgcc tcttccatgg gcccagacct gagcagaacc gggcttaccc 2580
   agctgcccag agagactcta ggggccagaa gccatgcccc agagctccca ggcagggctg 2640
   ccacccggga acttctgctt caagcttcag ggtagacaca gagacctgga tgagactcaa 2700
   gtcccctccc tgatcctggg cctgctgaag ccccttcctc catggctcct gtcatgagag 2760
   caaaacattg tcgcctggag acggtgactc ggaaagcctc ctgggagaca ggaaaggcat 2820
   cgatgccaca gctccatcct ctacttgacc cttgctggcg ggaggggagt gatatgtcca 2880
   cacactgcac tgcgtcatcc tgttccggat gcctccggag agagggacgg acagtcagaa 2940
   acaagggagt ttctattaaa ggccatccaa accaaaaaaa aaaaaaaaaa aa 2992
<210> 5
   <211> 727
   <212> PRT
   <213> horse
<400> 5
   Met Arg Pro Leu Leu Leu Leu Ala Pro Leu Gly Trp Leu Leu Leu Ala
   1               5                   10                  15
   Glu Ala Lys Gly Asp Ala Lys Pro Glu Asp Asn Leu Leu Val Leu Thr
               20                  25                  30
   Val Ala Thr Lys Glu Ser Glu Gly Phe Arg Arg Phe Lys Arg Ser Ala
           35                  40                  45
   Gln Phe Phe Asn Tyr Lys Ile Gln Ala Leu Gly Leu Gly Glu Asp Trp
       50                  55                  60
   Asp Gly Asp Lys Glu Thr Ser Ala Gly Gly Gly Leu Lys Val Arg Leu
   65                  70                  75                  80
   Leu Lys Lys Ala Leu Glu Lys His Ala Asp Lys Glu Asn Leu Val Ile
                   85                  90                  95
   Leu Phe Thr Asp Ser Tyr Asp Val Val Phe Ala Ser Gly Pro Arg Glu
               100                 105                 110
   Leu Leu Lys Lys Phe Arg Gln Ala Arg Ser Gln Val Val Phe Ser Ala
           115                 120                 125
   Glu Glu Leu Ile Tyr Pro Asp Arg Arg Leu Glu Ala Lys Tyr Pro Val
       130                 135                 140
   Val Ser Asp Gly Lys Arg Phe Leu Gly Ser Gly Gly Phe Ile Gly Tyr
   145                 150                 155                 160
   Ala Pro Asn Leu Ser Lys Leu Val Ala Glu Trp Glu Gly Gln Asp Ser
                   165                 170                 175
   Asp Ser Asp Gln Leu Phe Tyr Thr Lys Ile Phe Leu Asp Pro Glu Lys
               180                 185                 190
   Arg Glu Arg Ile Asn Ile Thr Leu Asp His Arg Cys Arg Ile Phe Gln
           195                 200                 205
   Asn Leu Val Gly Ala Leu Asp Glu Val Val Leu Lys Phe Glu Met Gly
       210                 215                 220
   His Val Arg Ala Arg Asn Leu Ala Tyr Asp Thr Leu Pro Val Leu Ile
   225                 230                 235                 240
   His Gly Asn Gly Pro Thr Lys Leu Gln Leu Asn Tyr Leu Gly Asn Tyr
                   245                 250                 255
   Ile Pro Arg Phe Trp Thr Phe Glu Thr Gly Cys Thr Val Cys Asp Glu
               260                 265                 270
   Gly Leu Arg Ser Leu Lys Gly Ile Gly Asp Glu Ala Leu Pro Val Val
           275                 280                 285
   Leu Val Gly Val Phe Ile Glu Gln Pro Thr Pro Phe Leu Ser Leu Phe
       290                 295                 300
   Phe Gln Arg Leu Leu Arg Leu His Tyr Pro Arg Lys Gln Leu Arg Leu
   305                 310                 315                 320
   Phe Ile His Asn His Glu Gln His His Lys Ala Gln Val Glu Gln Phe
                   325                 330                 335
   Leu Ala Glu His Gly Gly Glu Tyr Lys Ser Val Lys Leu Val Gly Pro
               340                 345                 350
   Glu Val Arg Val Ala Asn Ala Asp Ala Arg Asn Met Gly Ala Asp Leu
           355                 360                 365
   Cys Arg Gln Asp Arg Gly Cys Thr Tyr Tyr Phe Ser Val Asp Ala Asp
       370                 375                 380
   Val Ala Leu Thr Glu Pro Lys Thr Leu Arg Leu Leu Ile Glu Gln Asn
   385                 390                 395                 400
   Lys Asn Val Ile Ala Pro Leu Met Thr Arg His Gly Arg Leu Trp Ser
                   405                 410                 415
   Asn Phe Trp Gly Ala Met Ser Ala Asp Gly Tyr Tyr Ala Arg Ser Glu
               420                 425                 430
   Asp Tyr Val Asp Ile Val Gln Gly Arg Arg Val Gly Val Trp Asn Val
   435 440 445
   Pro Tyr Ile Ser Asn Ile Tyr Leu Ile Lys Gly Ser Ala Leu Arg Ala
   450 455 460
   Glu Leu Gln Gln Thr Asp Leu Phe His His Ser Lys Leu Asp Ala Asp
   465 470 475 480
   Met Ala Phe Cys Ala Asn Ile Arg Gln Gln Asp Val Phe Met Phe Leu
   485 490 495
   Thr Asn Arg His Thr Phe Gly His Leu Leu Ser Leu Asp Ser Tyr Gln
   500 505 510
   Thr Thr His Leu His Asn Asp Leu Trp Glu Val Phe Ser Asn Pro Glu
   515 520 525
   Asp Trp Lys Glu Lys Tyr Ile His Glu Asn Tyr Thr Arg Ala Leu Ala
   530 535 540
   Gly Lys Leu Val Glu Met Pro Cys Pro Asp Val Tyr Trp Phe Pro Ile
   545 550 555 560
   Phe Thr Glu Thr Ala Cys Asp Glu Leu Val Glu Glu Met Glu His Tyr
   565 570 575
   Gly Gln Trp Ser Leu Gly Asp Asn Lys Asp Asn Arg Ile Gln Gly Gly
   580 585 590
   Tyr Glu Asn Val Pro Thr Ile Asp Ile His Met Asn Gln Ile Ser Phe
   595 600 605
   Glu Arg Glu Trp His Lys Phe Leu Val Glu Tyr Ile Ala Pro Met Thr
   610 615 620
   Glu Lys Leu Tyr Pro Gly Tyr Tyr Thr Arg Ala Gln Phe Asp Leu Ala
   625 630 635 640
   Phe Val Val Arg Tyr Lys Pro Asp Glu Gln Pro Ser Leu Met Pro His
   645 650 655
   His Asp Ala Ser Thr Phe Thr Val Asn Ile Ala Leu Asn Arg Val Gly
   660 665 670
   Val Asp Tyr Glu Gly Arg Gly Cys Arg Phe Leu Arg Tyr Asn Cys Ser
   675 680 685
   Ile Arg Ala Pro Arg Lys Gly Trp Thr Leu Met His Pro Gly Arg Leu
       690                 695                 700
   Thr His Tyr His Glu Gly Leu Pro Thr Thr Lys Gly Thr Arg Tyr Ile
   705                 710                 715                 720
   Ala Val Ser Phe Val Asp Pro
                   725
<210> 6
   <211> 891
   <212> DNA
   <213> horse
<220>
   <221> misc_feature
   <222> (292)..(292)
   <223> n is a, c, g, or t
<220>
   <221> misc_feature
   <222> (552)..(552)
   <223> n is a, c, g, or t
<210> 7
   <211> 891
   <212> DNA
   <213> horse
<400> 7
   ggcaggggct gtcggttcct gcgttacaac tgctccatcc gagccccacg gaagggctgg      60
   accctcatgc accccggacg actcacgcac taccacgagg gactccccac caccaagggc     120
   acccgctaca tcgcggtctc cttcgtcgat ccctaatcgg ccaagcctgg ccacttcgga     180
   ccttttcctc gttgccaaca accactgccc agcagcctct ggggccttgg ggtcccaggg     240
   aacctggtcc agcctccggg ctcttgacct cccattgctt tcggagccgc catcggagag     300
   actgggccgc aggccagagg cagagcacac ctccttggct ggggctttcc tggtgttctg     360
   ctccccaccc cgggagatgg ggtccacgct cactgccttg taacagctca tcctctccca     420
   cctgttctcc tgaaaagccc ggtccctctt cctctgcctc ttccatgggc ccagacctga     480
   gcagaaccgg gcttacccag ctgcccagag agactctagg ggccagaagc catgccccag     540
   agctcccagg cagggctgcc acccgggaac ttctgcttca agcttcaggg tagacacaga     600
   gacctggatg agactcaagt cccctccctg atcctgggcc tgctgaagcc ccttcctcca     660
   tggctcctgt catgagagca aaacattgtc gcctggagac ggtgactcgg aaagcctcct     720
   gggagacagg aaaggcatcg atgccacagc tccatcctct acttgaccct tgctggcggg     780
   aggggagtga tatgtccaca cactgcactg cgtcatcctg ttccggatgc ctccggagag     840
   agggacggac agtcagaaac aagggagttt ctattaaagg ccatccaaac c              891
<210> 8
   <211> 130
   <212> DNA
   <213> horse
<400> 8
   agtttccagc ctgccagcgc ctgctggctg cccggacgtc cccagacctc ggccatgcgg      60
   cctctgctgc tcctggcccc gctgggctgg ctgctcctgg ccgaagcgaa gggcgacgcc     120
   aagccggagg                                                            130
<210> 9
   <211> 91
   <212> DNA
   <213> horse
<400> 9
   acaacctctt agtcctcacg gtggccacga aggagtccga ggggttccga cgcttcaagc      60
   gctcagccca gttcttcaac tacaagatcc a                                     91
<210> 10
   <211> 135
   <212> DNA
   <213> horse
<400> 10
   ggcgctgggc ctgggggagg actgggacgg ggacaaggag acgtcagcgg gcggcgggct        60
   gaaggttcgg ctgctgaaga aagctctgga gaagcatgca gacaaagaga acctggtcat       120
   tctcttcaca gacag                                                        135
<210> 11
   <211> 164
   <212> DNA
   <213> horse
<400> 11
   ctatgatgtg gtatttgcct cggggccccg agagctcctg aagaagttcc ggcaggccag        60
   gagccaggtg gtcttctcgg ccgaggagct catctacccc gaccgcaggc tggaggccaa       120
   gtacccggtg gtgtccgatg gcaagaggtt cctgggctct ggag                        164
<210> 12
   <211> 113
   <212> DNA
   <213> horse
<400> 12
   gcttcatcgg ttatgccccc aacctcagca aactggtggc tgagtgggag ggccaggaca        60
   gcgacagtga ccagctgttt tataccaaga tcttcttgga cccagagaag agg              113
<210> 13
   <211> 64
   <212> DNA
   <213> horse
<400> 13
   gagcggatca acatcaccct ggaccaccgc tgccgtatct tccagaacct ggttggagcc        60
   ttag                                                                     64
<210> 14
   <211> 98
   <212> DNA
   <213> horse
<400> 14
   atgaggtcgt gctcaagttt gaaatgggcc atgtgagggc gcggaacctg gcctacgaca 60
   ccctccccgt cctgattcat ggcaacgggc ccaccaag 98
<210> 15
   <211> 102
   <212> DNA
   <213> horse
<400> 15
   ctgcagctga actacctggg caactatatc cctcgcttct ggaccttcga gacgggctgc 60
   acggtgtgtg acgagggcct gcgcagcctc aagggcattg gg 102
<210> 16
   <211> 132
   <212> DNA
   <213> horse
<400> 16
   gatgaagctc tgcctgtggt cttggtcggc gtgttcatcg agcagcccac gccgttcctg 60
   tccctgttct tccagcggct tctgcgcctg cattaccccc ggaaacagct gcggcttttt 120
   attcataacc at 132
<210> 17
   <211> 122
   <212> DNA
   <213> horse
<400> 17
   gagcagcacc acaaggctca ggtggagcag ttcctggcag agcatggcgg cgagtacaag 60
   tctgtgaaac tggtgggccc cgaggtgcgg gtggcaaacg ccgatgccag gaacatgggc 120
   gc 122
<210> 18
   <211> 105
   <212> DNA
   <213> horse
<400> 18
   ggacctgtgc cggcaggacc gtggctgcac ctactacttc agtgtggatg ccgacgtggc 60
   cctgaccgag cccaagaccc tgcgactgct gattgagcag aacaa 105
<210> 19
   <211> 126
   <212> DNA
   <213> horse
<400> 19
   gaatgtcatc gccccgttga tgacccgcca cgggaggctg tggtcgaact tctggggggc 60
   aatgagtgca gatggctact acgcccgctc cgaggactac gtggacattg tgcaggggcg 120
   gcgtgt 126
<210> 20
   <211> 142
   <212> DNA
   <213> horse
<400> 20
   tggcgtctgg aacgtgccct acatctcgaa catttacctg atcaagggca gtgccctgcg 60
   ggctgagctg cagcagacag atctgttcca ccacagcaag ctggatgccg acatggcctt 120
   ctgtgccaat atccggcagc ag 142
<210> 21
   <211> 117
   <212> DNA
   <213> horse
<400> 21
   gatgtgttca tgttcctgac caaccggcac accttcggcc acctgctctc cctggacagc 60
   taccagacca cccacctcca caacgacctc tgggaggtgt ttagcaaccc cgaggac 117
<210> 22
   <211> 63
   <212> DNA
   <213> horse
<400> 22
   tggaaggaga agtacatcca tgagaactac accagggccc tggcggggaa gctggtggag 60
   atg 63
<210> 23
   <211> 105
   <212> DNA
   <213> horse
<400> 23
   ccttgcccgg atgtctactg gttccccatc ttcacggaga cggcctgtga cgagctggtg 60
   gaggagatgg agcactacgg ccagtggtct ctgggagaca ataag 105
<210> 24
   <211> 147
   <212> DNA
   <213> horse
<400> 24
   gacaaccgca tccagggtgg ctacgaaaat gtgccgacca tcgacatcca catgaaccag 60
   atcagctttg agcgggagtg gcacaagttc ctggtggagt acatcgcccc catgacagag 120
   aagctgtatc caggctacta caccagg 147
<210> 25
   <211> 126
   <212> DNA
   <213> horse
<400> 25
   gcccagttcg acctggcctt tgttgtccgc tacaagcctg acgagcagcc ctcgctgatg 60
   ccccaccacg atgcctccac cttcactgtc aacatcgccc tgaaccgggt cggggtggat 120
   tacgag 126
<210> 26
   <211> 350
   <212> DNA
   <213> horse
<400> 26
   atagctgtca ctccacaagg cacaagggct gcttggctgg ggtggctcag atgggagaat 60
   ggggggtact aggggaaggg cccagcttcc tcttggggaa actgacgctt cctgttggga 120
   aactgacact tcctgtctcc cagggcgggg gctgtcggtt cctgcgttac aactgctcca 180
   tccgagcccc acggaagggc tggaccctca tgcaccccgg acgactcacg cactaccacg 240
   agggactccc caccaccaag ggcacccgct acatcgcggt ctccttcgtc gatccctaat 300
   cggccaagcc tggccacttc ggaccttttc ctcgttgcca acaaccactg 350
<210> 27
   <211> 24
   <212> DNA
   <213> horse
<400> 27
   atagctgtca ctccacaagg caca 24
<210> 28
   <211> 25
   <212> DNA
   <213> horse
<400> 28
   cagtggttgt tggcaacgag gaaaa 25

## Description

This application claims priority to U.S. patent application serial no. 61/486,464, filed on May 16, 2011.

### FIELD OF THE INVENTION

The present invention relates generally to inherited disease observed in horses. More particularly, the invention relates to detecting a point mutation in the equine lysyl hydroxylase 1 (LH1) gene associated with Fragile Foal Syndrome Type 1.

### DESCRIPTION OF RELATED ART

Inherited connective tissue disorders occur in a variety of species. They are known by many names and are associated with many different underlying genetic defects. Nonetheless, they all present with skin hyperextensibility. Warmblood Fragile Foal Syndrome Type 1 (WFFST1) occurs in the Warmblood horse population which are a group of mid-sized horse types primarily originating in Europe, developed with the aim of competing in Olympic equestrian sports (often called Sport Horses). The presently available pedigree data shows that Warmblood Fragile Foal Syndrome Type 1 segregates in the Hanoverian, Selle Francais, KWPN (Dutch), Oldenburg, and Westphalian lines. These registries, as well as the Holsteiner, Wurtemberger, Rhinelander, Gelderlander, Zweibrucker and Bavarian Warmblood are central to all Warmblood/Sport Horse registries, placing all horses among the broader group of registries at risk for carrying this disorder. A partial list of many of the registries includes: Hanoverian; Selle Francais; KWPN; Oldenburg; Rhinelander; Holsteiner; Westphalian; Gelderlander; American Warmblood; Anglo-Norman; Austrian Warmblood; Bavarian Warmblood; Belgian Warmblood (including Belgian Half-blood); Canadian Sport Horse; Danish Warmblood; Brazilian Sport Horse; Friesian Sport Horse; German Warmblood ZfDP; Czech Warmblood; Irish Sport Horse; Karossier/Ostfriesen/Alt-Oldenburger; Hungarian Warmblood; Romanian Sport HorseSwedish Warmblood; Swiss Warmblood; Wurttemberger; and Zweibrucker. There is an ongoing an unmet need to identify the mutation that causes WFFST1 and to develop compositions and methods for determining which horses carry it and which horses do not. The present invention meets these and other needs.

### SUMMARY OF THE INVENTION

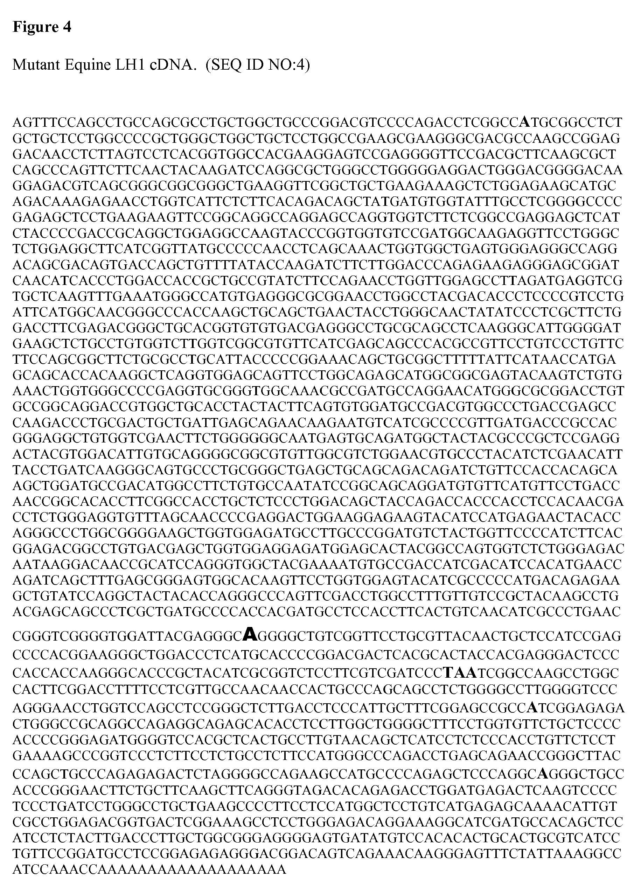

The present invention is based on the discovery of the mutation associated with WFFST1. The method comprises testing a biological sample obtained or derived from a horse to identify the presence of a G to A mutation in the equine lysyl hydroxylase 1 (LH1) gene. The location of the change will be apparent to those skilled in the art from the amino acid and nucleotide sequences described herein. In one embodiment, the mutation is at a nucleotide position 2086 of the cDNA produced using mRNA transcribed from an LH1 gene which contains the mutation. This cDNA is presented in Figure 4 as SEQ ID NO:4. Determining a G to A mutation in only one allele establishes that the horse is heterozygous for the WFFST1 mutation and can thus be identified as a carrier. Determining the G to A mutation in both alleles establishes that the horse is homozygous for the WFFST1 mutation and can thus be identified as affected or predisposed to developing WFFST1. Determining an absence of the G to A mutation in both alleles establishes that the horse is homozygous for the absence of the allele and is indicative that the horse is genetically normal with respect to WFFST1.

The mutation can be determined from DNA or RNA, or from a cDNA amplified from RNA, or from protein obtained from a horse. In one embodiment, the presence of the mutation is identified by determining the presence of a polynucleotide comprising the sequence of SEQ ID NO:1, which is a PCR product obtained by amplification of a segment of the LH1 gene from a horse, the genome of which comprised the mutation. In another embodiment, determining the presence of the mutation comprises determining a change from G to A at position 4 in the sequence of SEQ ID NO:6, which is the wild type sequence of exon 19 of the equine LH1 gene. The sequence of exon 19 comprising the mutation is presented as SEQ ID NO:7.

In various embodiments, determining the presence of the WFFST1 mutation (irrespective of heterozygosity or homozygosity for it) establishes that the horse is not genetically normal with respect to WFFST1. Determining the absence of the mutation (irrespective of heterozygosity or homozygosity for its absence) establishes that the horse is not affected with WFFST1.

Also provided is a method for selecting horses for breeding. This aspect comprises obtaining a biological sample from a horse, testing the biological sample to detect the WFFST1 mutation and, based on analysis of the allelic status for the WFFST1 mutation, breeding horses that are genetically normal or heterozygous for it with horses that are genetically normal with respect to WFFST1.

Isolated nucleic acids which comprise the mutation are also provided by the invention.

### BRIEF DESCRIPTION OF THE DRAWINGS

Figure 1A provides a photographic representation of excessive skin elasticity that is typical of horses affected with WFFST1.
Figure 1B provides a photographic representation of a skin lesion that is typical of horses affected with WFFST1.

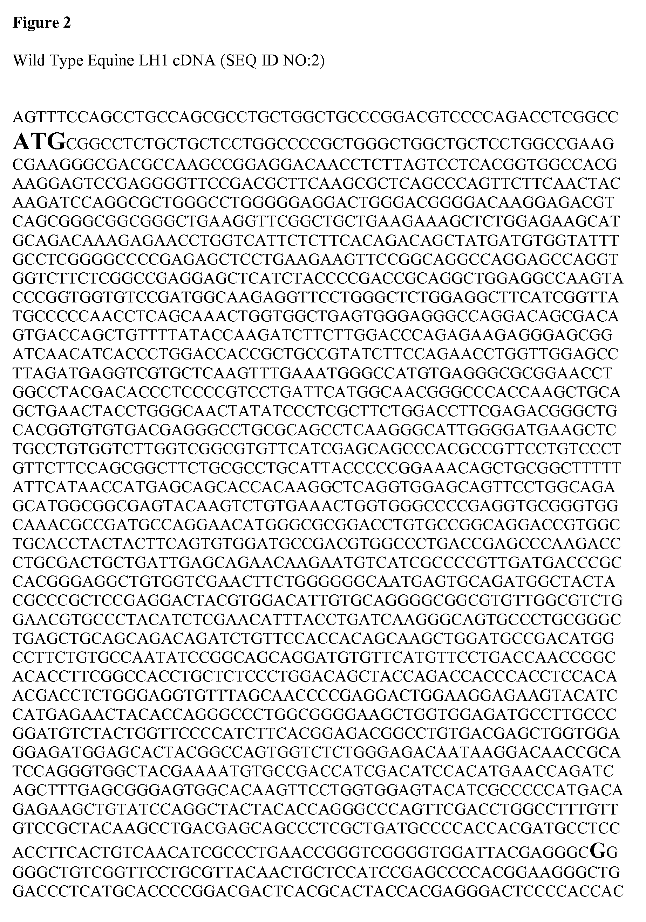

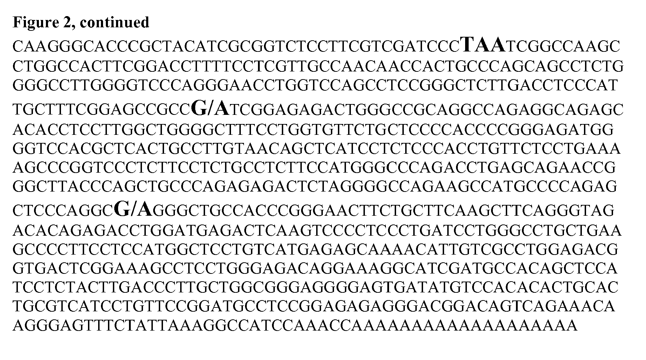

Figure 2 provides the sequence of wild type equine LH1 cDNA (SEQ ID NO:2). The wild type bases at the positions of the mutation (base 2086) is bold and enlarged. Non-informative non-coding region single nucleotide polymorphisms (SNPs) are located at bases 2320 and 2580 (when the A of the ATG translational start codon beginning at position 55 is designated as nucleotide 1). Either G or A may occur at these positions in wild-type alleles. Thus there are two additional nucleotides depicted in this sequence in Figure 2 than would exist in the cDNA, which is for convenience of illustrating this variability. In the depicted sequence the A of the ATG codon is at position 55. The translational stop codon is position 2182-2184 when designating the A at position 55 as nucleotide number 1.

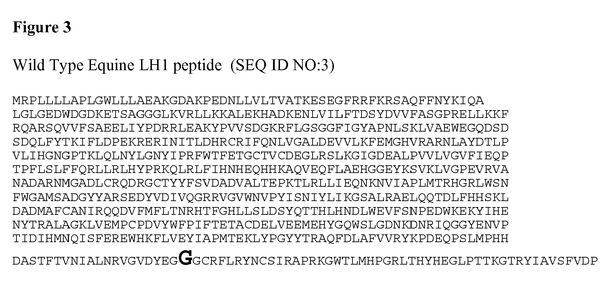

Figure 3 provides the wild type equine LH1 polypeptide sequence (727 amino acids; SEQ ID NO:3). The position of the amino acid in the normal peptide corresponding to the position of the change in mutation is bold and enlarged.
Figure 4 provides the sequence of a mutant equine LH1 cDNA (SEQ ID NO:4). The position of the mutation is 2032 (when the A at position 55 is designated nucleotide 1), and non-informative, non-coding region SNPs are bold and enlarged. These are respectively at 2320 and 2580. The A of the ATG codon at 55 is bold and enlarged. The translational stop codon is position 2182-2184, and is bold and enlarged. When the first nucleotide of the sequence is counted as nucleotide number 1, the mutation is at position 2086 and the other features of the sequence described above can take their numerical positions in relation to this numbering convention.

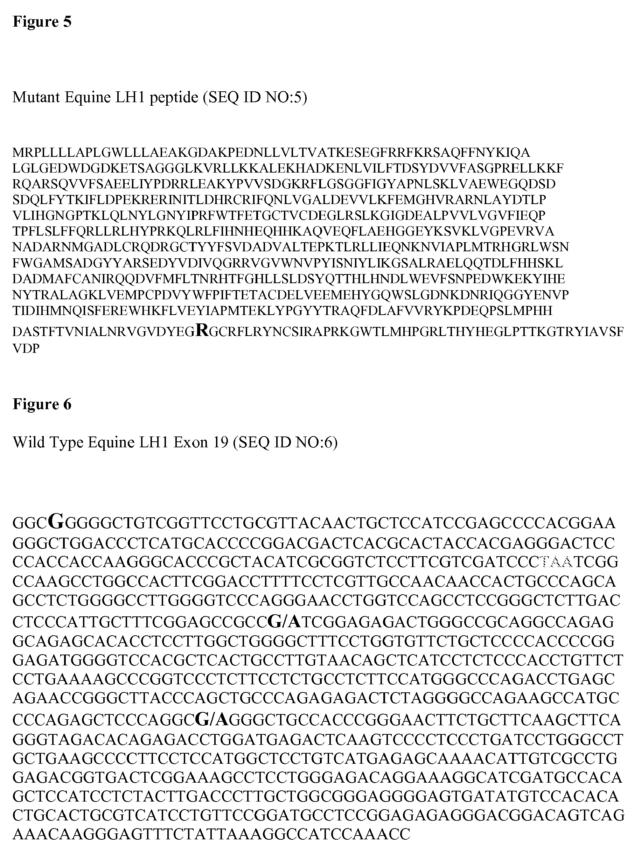

Figure 5 provides the amino acid sequence of the mutant equine LH1 peptide (SEQ ID NO:5). Arginine replaces the evolutionarily conserved Glycine at position 678 (R is bold and enlarged).
Figure 6 provides the sequence of the wild type equine LH1 exon 19 (SEQ ID NO:6). The wild type base at the position of the mutation (base 4) is bold and enlarged and non-coding region SNPs are also shown in bold and enlarged. These are respectively at bases 292 and 552, and either G or an A may occur at these positions in wild-type alleles, while an A occurs at each of these positions in the WFF 1 mutant allele. The translational stop codon is bold and enlarged.

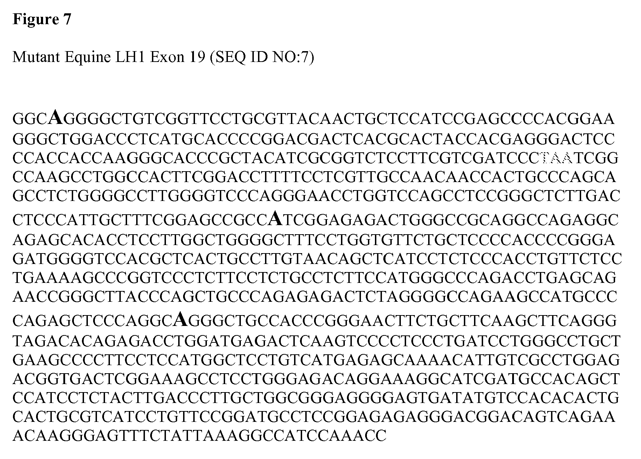

Figure 7 provides the sequence of the mutated equine LH1 exon 19 (SEQ ID NO:7). The mutation (base 4) is shown in bold and enlarged. The non-informative non-coding region SNPs are also bold and enlarged and occur at bases 292 and 552. The translational stop codon is shown in bold and is enlarged.

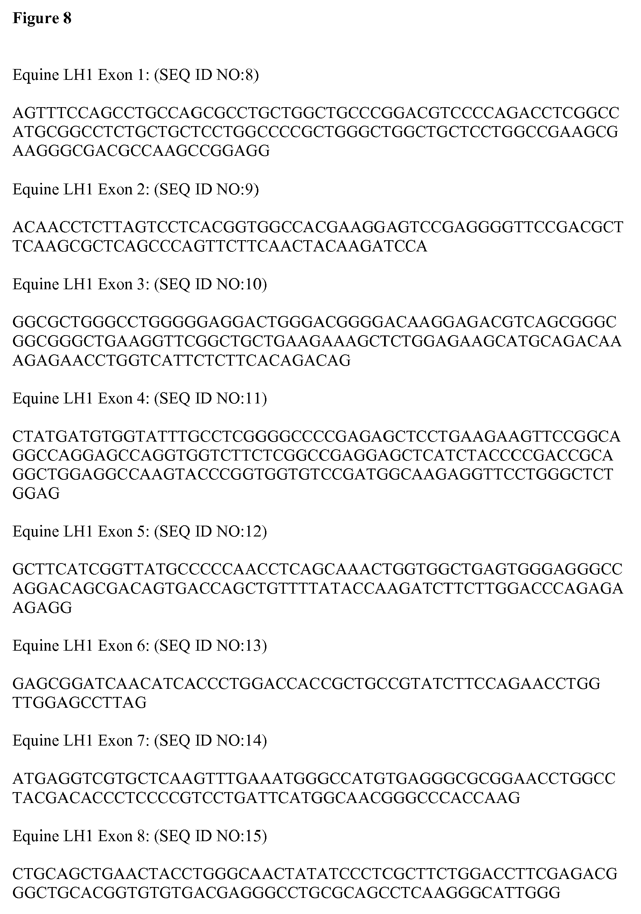

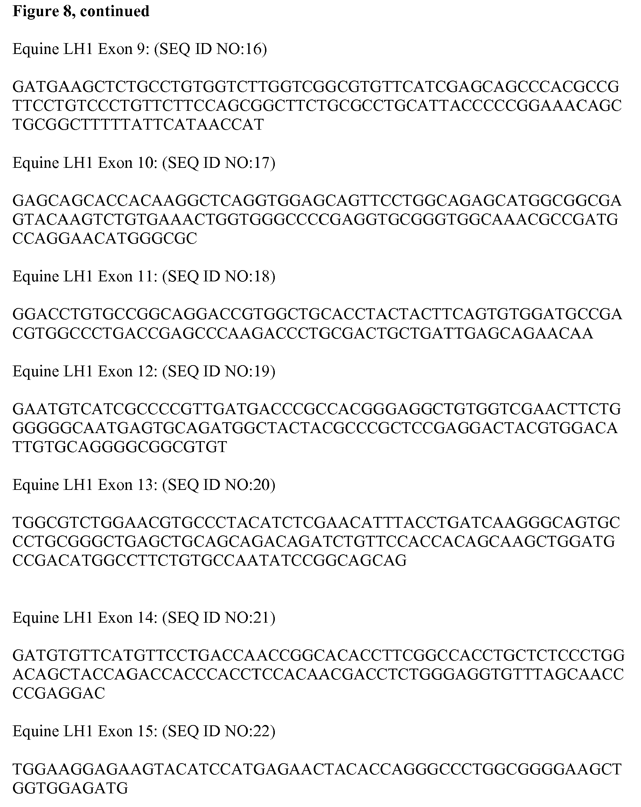

Figure 8 provides the sequences of exons 1-18 of the equine LH1 gene (SEQ ID NOs 8-25, respectively).
Figure 9A provides the nucleotide sequence of a representative amplicon obtained by PCR amplification of an equine LH1 gene that does not contain the mutation (SEQ ID NO:26).
Figure 9B provides the nucleotide sequence of a representative amplicon obtained by PCR amplification of an equine LH1 gene that does contain the mutation. The mutation is at position 147 and is enlarged and shown in bold (SEQ ID NO:1).

### DETAILED DESCRIPTION OF THE INVENTION

The present invention is based on the discovery of a mutation which is positively correlated with WFFST1. The mutation is a G to A change in the equine LH1 gene. The mutation is referred to herein as the "WFFST1" mutation. The invention comprises a method for analysis of WFFST1 status in a horse. The method comprises obtaining a biological sample from a horse and testing nucleic acids and/or proteins from the biological sample to determine the presence or absence of the WFFST1 mutation. Determining a G to A mutation in only one allele establishes that the horse is heterozygous for the WFFST1 mutation. The horse can thus be identified as a carrier of WFFST1. Determining the G to A mutation in both alleles establishes that the horse is homozygous for the WFFST1 mutation. The horse can thus be identified as affected or predisposed to developing WFFST1. Determining an absence of the G to A mutation in both alleles establishes that the horse is homozygous for the absence. The horse can accordingly be identified as genetically normal with respect to WFFST1. An LH1 allele that does not comprise the WFFST1 mutation and is thus genetically normal with respect to WFFST1 is also referred to herein as "wild type." In one embodiment of the invention, determining the presence of a wild type WFFST1 allele establishes that the horse is not affected or predisposed to developing WFFST1. In another embodiment, determining the presence of a WFFST1 mutation establishes that the horse is not genetically normal with respect to WFFST1.

The location of the G to A change in any particular sample of equine nucleic acids can be ascertained by the skilled artisan via reference to the nucleotide sequences presented herein. The invention encompasses detecting the presence or absence of the WFFST1 mutation in any polynucleotide from any suitable biological sample obtained or derived from a horse. Detecting the mutations by testing the polypeptide encoded by the LH1 gene is also included. Further, for each DNA sequence described herein its RNA equivalent (replacing each T with a U) is included in the invention. All polynucleotides which encode the equine LH1 polypeptides presented herein are also included with the scope of the invention.

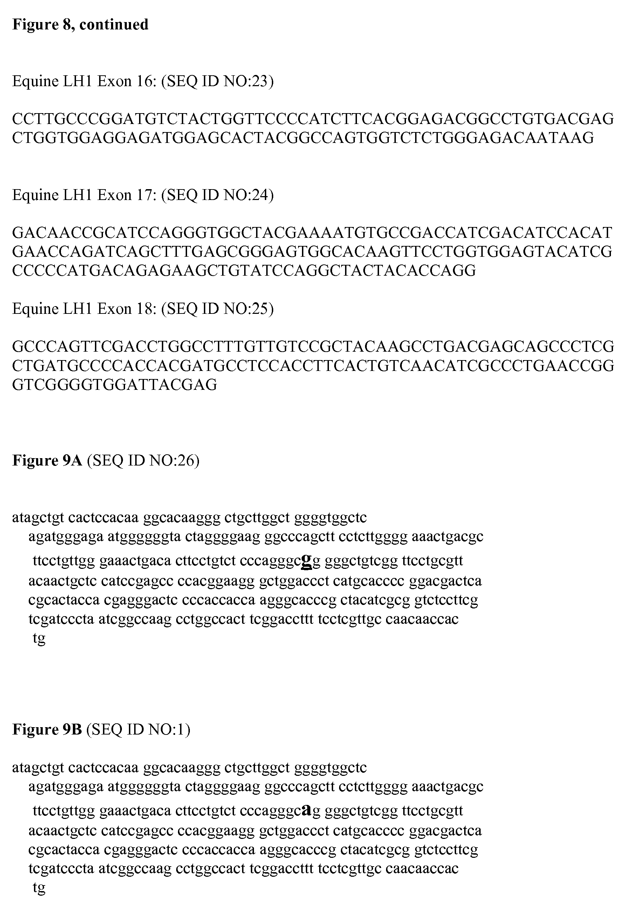

The WFFST1 mutation is shown in one illustrative embodiment as an A at position 147 of SEQ ID NO:1 (Fig. 9B). SEQ ID NO:1 is the sequence of an amplicon obtained by PCR amplification of a sample comprising nucleic acids which contained the mutation. The A at position 147 of SEQ ID NO:1 represents a change from a wild type G to the mutated sequence which comprises an A at position 147. The sequence of an amplicon obtained by PCR amplification of a sample which did not contain the mutation in presented in SEQ ID NO:26 (Fig. 9B).

Other polynucleotide sequences presented herein show the mutation in extended equine genetic sequences. For example, the WFFST1 mutation is also shown as an A at position 2086 in the sequence of SEQ ID NO:4. This WFFST1 mutation can be designated to be at position 2032 of SEQ ID NO:4 if nucleotide numbering in that sequence begins with the A at position 55, which is the A of the ATG translation initiation codon. SEQ ID NO:4 is the sequence of a cDNA produced from mRNA transcribed from an LH1 gene which contains the mutation. Non-informative, non-coding region SNPs are present at positions 2320 and 2580 (numbering of nucleotides as if the A of the ATG translational start codon is designated as nucleotide position 1). The non-informative SNPs can be A or G at these positions. The pertinent translational stop codon is position 2182-2184 (again where numbering using A at position 55 of the cDNA is designated as nucleotide position 1).

In another embodiment, determining the presence of the mutation comprises determining a change from G to A at position 4 in the sequence of SEQ ID NO:6. In connection with this, the wild type sequence of exon 19 of the equine LH1 gene as shown in SEQ ID NO:4 comprises G at position 4. The sequence of exon 19 comprising the WFFST1 mutation is provided in SEQ ID NO:7. Thus, the invention includes determining the presence of the mutation by determining the sequence of SEQ ID NO:7 from a sample obtained from a horse, or determining the absence of the mutation by determining the sequence of SEQ ID NO:6 from a sample obtained from a horse.

Those skilled in the art will recognize from the foregoing description that position 2086 in SEQ ID NO:2, position 2086 in SEQ ID NO:4, position 147 in SEQ ID NO:1, position 147 in SEQ ID NO:26, position 4 in SEQ ID NO:6, and position 4 in SEQ ID NO:7 are equivalent expressions of the location of the site where the G to A change that constitutes the WFFST1 mutation takes place at the genomic DNA level.

Those skilled in the art will also recognize that probes and primers can be developed for detecting LH1 sequences that contain the mutation or for LH1 sequences that do not contain the mutation based on the nucleotide sequences described herein. For example, any fragment of SEQ ID NO:2 or SEQ ID NO:4, or their complements, that is at least 12 nucleotides long and does not encompass the mutation site can be used as a primer for any conventional amplification or sequencing reaction to perform the method of the invention. Any fragment that is at least 12 nucleotides long and which does encompass the mutation site can be used for detecting the presence or absence of the mutation using any conventional technique that involves hybridization of a diagnostic probe that can discriminate between an A or a G at the mutation site. In specific embodiments, PCR can be performed on DNA obtained from a horse with a first primer that is identical or complementary to nucleotides 1-24 of SEQ ID NO:1 and a second primer which is identical or complementary to the 25 nucleotides at the 3' end of SEQ ID NO:1. Those skilled in the art will recognize that the sense orientation of the first and second primers will be opposite according to conventional PCR methods.

The WFFST1 mutation results in a change in the amino acid sequence encoded by the LH1 gene which comprises it. In particular, an equine LH1 polypeptide sequence encoded by an equine LH1 gene which contains the mutation is presented in SEQ ID NO:5. This polypeptide differs from the wild type sequence (a polypeptide sequence encoded by an equine LH1 gene that does not contain the mutation, depicted in SEQ ID NO:3) at amino acid position 678. In the mutant sequence the amino acid at position 678 is Arginine, while in the wild type sequence this amino acid position is occupied by Glycine. Thus, in various embodiments, the invention provides for testing LH1 protein from a biological sample obtained or derived from a horse to determine the amino acid at position 678. In one embodiment, detecting LH1 protein comprising Arginine at position 678 establishes that the horse that produced it is not genetically normal with respect to the WFFST1 mutation.

The invention can be carried out using any suitable biological sample obtained from a horse. In various embodiments, the biological sample contains nucleic acids encoding the LH1 protein or portions thereof, or a biological sample that contains the LH1 protein, or combinations of such polynucleotides and protein. Suitable sources of biological sample include but are not limited to blood, hair, mucosal scrapings, semen, tissue biopsy, or saliva. In one embodiment, the biological sample is blood. In one embodiment, the biological sample is obtained from the horse and used directly in analyzing the polynucleotides and/or protein. In another embodiment, the biological sample is obtained from the horse and subjected to a processing step before the analysis. In some examples, the processing step can be carried out to isolate or purify the polynucleotides and/or proteins to be analyzed, or to amplify the polynucleotides. Thus, the invention in certain embodiments comprises extracting a biological sample from a horse so that the sample is separated from its naturally occurring environment, and mixing it with a non-naturally occurring reagent, such as an extraction buffer. The sample can be mixed with organic solvents, such as formaldehyde, chloroform, alcohols, and the like. The sample may also be subjected to successive heating and cooling steps, or freezing temperatures, such as -20 Celsius.

While any suitable technique can be used to detect the presence or absence of the WFFST1 mutation, presently preferred techniques include polymerase-chain based reactions, such as PCR or real-time PCR (RT-PCR), or reverse-transcriptase based PCR, or restriction digestions. These processes involve well known techniques and generally include providing a composition comprising isolated nucleic acids for testing and mixing the nucleic acids with non-naturally occurring reagents, such as recombinant or isolated bacterial, viral or bacteriophage polymerases, buffers comprising pre-fixed concentrations of free deoxyribonucleotides, including those that are unlabeled and/or those that are detectably labeled, and/or recombinant bacterial restriction endonucleases, and combinations of the foregoing. The compositions can comprise one or more synthetic oligonucleotides that have complete or partial complementarity to the isolated equine polynucleotide so that they can function as probes, such as primers. In another embodiment, an isolated polynucleotide which comprises or might comprise the WFFST1 mutation can be annealed to a synthetic oligonucleotide that is fixed to a substrate, such as glass or a resin. For example, an isolated polynucleotide which comprises or is suspected of comprising the WFFST1 mutation can be annealed to a synthetic oligonucleotide that is present on an array which comprises a plurality of distinct oligonucleotides. Such arrays can distinguish polynucleotides that differ from one another by a single nucleotide and will produce a signal that is detectable by an individual and/or a machine to signify the presence or the absence of the mutation.

In another embodiment, the presence or the absence of the WFFST1 mutation in a polynucleotide can be determined using a restriction endonuclease digestion. For example, when present the WFFST1 mutation introduces a unique AlwN1 site. Using a polynucleotide consisting of SEQ ID NO:1 hybridized to its complement as a non-limiting example, digesting this amplicon with AlwN1 yields fragments of ∼ 150 and 200 bp on digestion, as compared to the uncut 350 bp fragment obtained when the wild-type allele is present. Furthermore, the WFFST1 mutation ablates an AciI site, yielding fragments of ∼269, 10 and 72 bp, while digestion of the wild-type fragment produces ∼ 149, 120, 10 and 72 bp bands in this illustrative exmaple. Thus, digestion with AciI cleaves the wild-type product in three places while the mutant product is only cleaved in two places. Those skilled in the art will recognize that there are a multitude of restriction endonuclease assays that can be developed if desired from these observations.

As with polynucleotides, detecting a protein which comprises the WFFST1 mutation can be performed using any known technique. Such techniques include but are not limited to immunodetction assays, which entail forming a complex between a protein that comprises or might comprise the amino acid change encoded by the WFFST1 mutation with a monoclonal antibody that is produced by a hybridoma or by recombinant methods. The complex can include a detectably labeled ligand, such as a detectably labeled monoclonal antibody that is specific for the protein. The invention includes generation and use of monoclonal antibodies that can discriminate between the wild type and mutant forms of the LS1 protein.

Also provided in the present invention are kits for detecting the presence or absence of the WFFST1 mutation. The kits comprise reagents for nucleic acid based detection of the presence or absence of the mutation(s), or antibodies for detecting the presence or absence of the mutation. In one embodiment, the kits comprise reagents for extraction/preparation of nucleic acid samples and pair(s) of specific primers for use in identification of the mutation. In another embodiment, the kits provide antibodies and compositions used for probing samples with the antibodies to determine whether or not the WFFST1 mutation is present in a horse from which a biological sample is obtained and tested according to the invention. Any of the aforementioned kit components can be provided in one or more sealed, sterile containers, such as vials.

It is contemplated that any horse at any developmental stage (i.e., fetal, neonatal, postnatal, juvenile, adult, etc.) may be tested according to the method of the invention. Further, any horse, regardless of breed, could be homozygous or heterozygous for the WFFST1 mutation, or homozygous for the absence of the mutation, and thus is suitable for testing. In various embodiments, the horse may be a Warmblood, Thoroughbred, Standardbred, Tennessee Walking Horse, Mustang, Quarter Horse, Arabian, Draft breed, Minature Horse, or a pony.

By using the tools and method described herein, horses which are genetically normal for the disease, carriers of the WFFST1 disease and horses which are affected by (or predisposed to) WFFST1 can be identified. Upon identification, affected or predisposed, or carrier horses can be eliminated from the breeding stock. Alternatively, horses which are affected or predisposed with WFFST, or carriers of the WFFST mutation, can be mated with genetically normal horses to avoid producing affected foals.

In one embodiment, the method further comprises fixing the result of determining the presence or absence of the WFFST1 mutation in a tangible medium, such as a paper report, or a portable medium on which electronic files can be stored, such as a hard drive, a computer disk, a thumb drive, and the like. In another embodiment, the invention includes communicating the result of determining the presence or absence of the WFFST1 mutation to an individual. The test result can be communicated to an individual by any method. Non-limiting examples of the individual to whom the test results may be communicated include the horse owner, an equine registry, a veterinarian, or a provider of genetic test results.

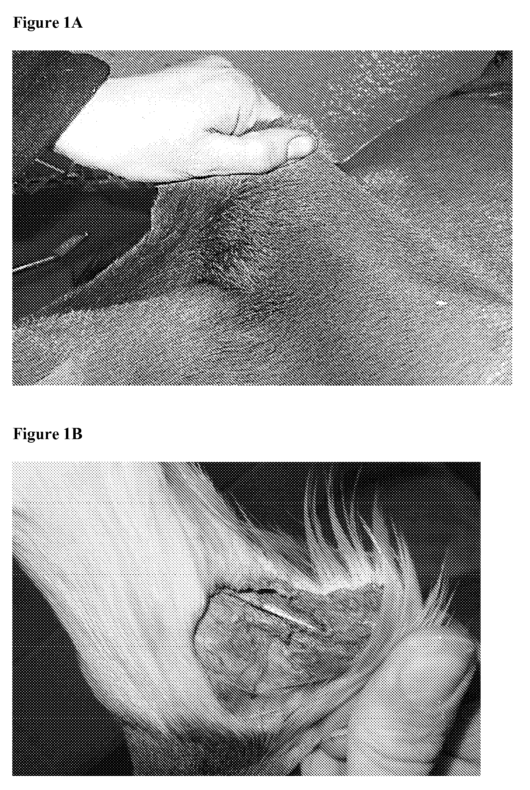

The WFFST1phenotype is clinically detectable and present at birth (premature birth has been reported). Affected foals are euthanized within days to weeks of life due to the poor prognosis. Horses that are clinically affected can be recognized by skin traits that are characteristic of WFFST1. Figs 1A and 1B illustrate typical manifestations of WFFST1, where the skin lacks tensile strength (Fig. 1A) and extreme skin fragility characterized by tearing, ulceration, etc. from contact with normal surroundings; Fig 1B). Lesions can occur anywhere on the body, but are most noted on pressure points and in addition to skin wounds, lesions are found on the gums and other oral cavity mucous membranes and the perineum. Skin is hyperextensible (stretchy) over essentially the whole body, and especially the head, neck, thorax and limbs. Limb joints are lax and hyperextensible. Fetlocks are the most dramatically affected and affected foals cannot stand normally. Ears may be soft, overly flexible, bent or floppy presumably due to failure of collagen in the supporting cartilage. At post mortem exam hematomas and seromas are noted under the skin, particularly over pressure points. Supporting structures around joints (capsule, tendons, ligaments) are lax. Lesions of articular cartilage are reported in some joints.

The invention will be further understood by the following Example, which is intended to be illustrative and not restrictive in any way.

### Example 1

This Example demonstrates a 100% correlation between homozygosity (AA) for the causative mutation and the disease phenotype. The disease phenotype is recognizable on clinical examination and is characterized by the presence at birth of soft skin lacking adequate tensile strength to withstand even normal environmental contact. The skin is hyperextensible (lacking normal recoil when traction is applied/released) over the whole body, especially the head, neck, thorax and limbs. Over the first hours to days of life, focal seromas, hematomas, and lacerations/ulcerations result from incidental environmental contact. Such lesions are predominantly found over pressure points such as fetlocks (metacarpal-phalangeal joints), carpi (knees), and tarsi (hocks), but may occur anywhere on the body (including oral cavity and perineum). Limb joints, particularly the fetlocks) are lax and hyperextensible, and affected foals cannot typically stand normally. The ears are typically bent or floppy. Also demonstrated is a 100% correlation between heterozygosity (AG) and the normal phenotype, as well as a 100% correlation between homozygosity for wild type (GG) and the normal phenotype. The normal phenotype lacks the clinical criteria specified above for the disease phenotype.

Results from determining genotype and phenotype are presented in Table 1. To obtain the results presented in Table 1, the WFFST1 genotype was established by PCR amplification of a 350 bp fragment of the LH1 gene containing the WFFST1 mutation site from genomic DNA, followed by automated direct sequencing of the resulting PCR products. The sequences of amplified fragments (amplicons) representing the wild-type and WFFST1 alleles are given in SEQ ID NO: 26 and SEQ ID NO: 1, respectively. Genomic DNA was extracted from whole blood/serum or mane/tail hair root samples using respectively, the QIAamp DNA Blood Mini Kit or the DNeasy Blood and Tissue Kit (QIAGEN) according to the manufacturer's recommended protocols. Amplifications were performed in 50 microliters total volume containing 3 microliters genomic DNA (~40 ng), 1 X Platinum Taq Hi Fi buffer, 200 micromolar dNTPs, 0.2 micromolar each primer, and 1 unit Platinum Taq Hi Fi (Invitrogen). Primer sequences for PCR were 5'-atagctgtcactccacaaggcaca-3' (sense primer; SEQ ID NO:27) and 5'-cagtggttgttggcaacgaggaaaa-3' (antisense primer; SEQ ID NO:28). DNA amplification was performed in an ABI 2720 thermal cycler using the following program: Initial denaturation: 5 min at 95°, followed by 40 cycles of: 30 sec at 95°; 30 sec at 55°; 30 sec at 72°; followed by a final extension segment of 5 min at 72°. Unincorporated primers and nucleotides were eliminated from PCR reactions using the DNA Clean & Concentrator-5 Kit (Zymo Research) according to the manufacturer's recommended protocol. DNA sequencing was performed by cycle sequencing using a primer having the sequence 5'-atagctgtcactccacaaggcaca-3', using dye terminator chemistry (Big Dye v3.1, Applied Biosystems, Foster City CA), followed by dye terminator removal using Edge Performa DTR gelfiltration plates (Edge Biosystems, Gaithersburg MD) and automated data collection on an Applied Biosystems 3730x1 DNA Analyzer.

The results presented in Table 1 demonstrate a 100% association between the presence of a G to A mutation in the LH1 gene at position 2086 in the sequence of SEQ ID NO:4. More specifically, there is a 100% association between homozygosity (A/A) for the causative mutation and the disease phenotype; a 100% association between heterozygosity (A/G) and the normal phenotype, as well as a 100% association between homozygosity for wild type (G/G) and the normal phenotype.

**Table 1**

| **Genotype** | Homozygous for the mutant allele (A/A) | Heterozygous for the mutant allele (A/G) | Homozygous for the normal allele (G/G) |
|---|---|---|---|
| **Number Tested** | 2 | 8 | 149 |
| **Clinical Phenotype** | 2 Affected | 8 Normal | 149 Normal |

The data summarized in Table 1 show that two foals homozygous for the mutant (A) allele were clinically affected. The phenotype of affected foals was determined by clinical examination over the initial days of life. Eight Warmblood horses were determined to be heterozygous for the mutant allele (A/G) and all eight were clinically normal. Three of the eight heterozygotes were obligate carrier parents of the affected foals. The five additional heterozygotes were identified by screening DNA samples from 104 clinically normal Warmbloods from the local population. One hundred and forty-nine horses were determined to be homozygous for the normal allele (G) and all were clinically normal. This group of horses included 50 horses of various breeds (Thoroughbred, Standardbred, Tennessee Walking Horse, Mustang, Quarter Horse, Arabian, Drafts and ponies) in addition to 99 clinically normal Warmbloods.

The results in Table 1 show clearly that there is a 100% association (2/2 horses) between homozygosity for the causative mutation and the affected phenotype. The frequency of the mutant allele (A) appears to be approximately 3.77% in the test population. The frequency of the mutant allele (A) in the "at-risk" population (Warmbloods) appears to be approximately 5.50%, while the carrier frequency among the group of clinically normal adult Warmbloods studied is 7.48%. Mutations in lysyl hydroxylase 1 (LH1) are known to cause the human disorder Ehlers-Danlos Syndrome VI (EDSVI). This is an autosomal recessive connective tissue fragility syndrome characterized by premature birth, infant floppiness/joint laxity, development of kyphosis and/or scoliosis, skin fragility, abnormal bruisability and scarring, ocular abnormalities, recurrent joint dislocation, cardiac valvular failure, catastrophic arterial and/or bowel rupture. The present invention is believed to represent the first discovery of a spontaneously occurring EDSVI analog in a non-human animal species, and mutations causative of deficient/abnormal LH1 are believed to have not previously been described in non-human animals.

## Claims

1. A method for determining whether a horse is heterozygous, homozygous or has an absence of a Warmblood Fragile Foal Syndrome Type 1 (WFFST1) mutation, wherein the WFFST1 mutation comprises a change from G to A at position 147 in the sequence of SEQ ID NO:26, the method comprising: determining from a biological sample from the horse the presence or absence of the WFFST1 mutation, and identifying the horse as WFFST1 mutation homozygous by determining homozygosity for the WFFST1 mutation; or identifying the horse as WFFST1 mutation heterozygous by determining heterozygosity for the WFFST1 mutation; or identifying the horse as genetically normal with respect to WFFST1 by determining a homozygous absence of the WFFST1 mutation.

2. The method of claim 1, wherein the determining the presence or absence of the WFFST1 mutation comprises isolating DNA or RNA from the biological sample and testing the isolated DNA and/or RNA for the presence or absence of the WFFST1 mutation.

3. The method of claim 1, wherein the determining the presence or absence of the WFFST1 mutation comprises processing the biological sample to isolate DNA which comprises or might comprise the WFFST1 mutation, subsequently amplifying a segment of the isolated DNA which comprises or might comprise the WFFST1 mutation to obtain a DNA amplification product, and testing the DNA amplification product to determine the presence or absence of the WFFST1 mutation.

4. The method of claim 3, wherein the determining the presence or absence of the WFFST1 mutation comprises separating RNA from the biological sample, creating a cDNA from the separated RNA, and testing the cDNA to determine the presence or absence of the WFFST1 mutation.

5. The method of claim 1, wherein the determining the presence or absence of the WFFST1 mutation comprises isolating protein from the biological sample and testing the isolated protein for the presence of a protein comprising the sequence of SEQ ID NO:5, wherein the presence of a protein comprising the sequence of SEQ ID NO:5 establishes the presence of the WFFST1 mutation.

6. A method for determining whether a genome of a horse comprises a Warmblood Fragile Foal Syndrome Type 1 (WFFST1) mutation, wherein the WFFST1 mutation comprises a change from G to A at position 4 in the sequence of SEQ ID NO:6, the method comprising: testing a biological sample from the horse to determine the presence or absence of the WFFST1 mutation.

7. The method of claim 6, wherein the determining the presence of the mutation comprises determining the presence of SEQ ID NO:7.

8. The method of claim 6, wherein the determining the absence of the mutation comprises determining the presence of SEQ ID NO:6.

9. The method of claim 6, comprising determining the absence of the WFFST 1 mutation and identifying the horse from which the biological sample was obtained as not affected with WFFST1.

10. The method of claim 6, comprising determining the presence of the WFFST1 mutation and identifying the horse from which the biological sample was obtained as not genetically normal with respect to WFFST1.

11. The method of claim 6, comprising determining homozygosity for the WFFST1 mutation and identifying the horse from which the biological sample was obtained as affected with or predisposed to WFFST1.

12. The method of claim 6, comprising determining heterozygosity for the WFFST1 mutation and identifying the horse from which the biological sample was obtained as a carrier of the WFFST1 mutation.

13. The method of claim 6, wherein the determining the presence of the WFFST 1 mutation comprises detecting from the biological sample a polypeptide comprising the sequence of SEQ ID NO:5.

14. A composition comprising an isolated nucleic acid, wherein the isolated nucleic acid comprises the sequence of SEQ ID NO:1.

## Patentansprüche

1. Verfahren zur Bestimmung, ob ein Pferd heterozygot, homozygot ist oder keine Warmblood Fragile Foal Syndrome Typ 1 (WFFST1)-Mutation aufweist, wobei die WFFST1-Mutation eine Änderung von G zu A in der Position 147 in der Sequenz von SEQ-ID-NR. 26 umfasst, wobei das Verfahren Folgendes umfasst: Bestimmen in einer biologischen Probe von dem Pferd der Gegenwart oder Abwesenheit der WFFST1-Mutation und Identifizieren des Pferdes als homozygot für die WFFST1-Mutation durch Bestimmen der Homozygotie für die WFFST1-Mutation; oder Identifizieren des Pferdes als heterozygot für die WFFST1-Mutation durch Bestimmen der Heterozygotie für die WFFST1-Mutation; oder Identifizieren des Pferdes als genetisch normal bezüglich WFFST1 durch Bestimmen einer homozygoten Abwesenheit der WFFST1-Mutation.

2. Verfahren nach Anspruch 1, wobei das Bestimmen der Gegenwart oder Abwesenheit der WFFST1-Mutation Folgendes umfasst: Isolieren von DNA oder RNA aus der biologischen Probe und Testen der isolierten DNA und/oder RNA auf die Gegenwart oder Abwesenheit der WFFST1-Mutation.

3. Verfahren nach Anspruch 1, wobei das Bestimmen der Gegenwart oder Abwesenheit der WFFST1-Mutation Folgendes umfasst: Verarbeiten der biologischen Probe, um DNA isolieren, die die WFFST1-Mutation umfasst oder umfassen könnte, anschließendes Amplifizieren eines Segments der isolierten DNA, die die WFFST1-Mutation umfasst oder umfassen könnte, um ein DNA-Amplifikationsprodukt zu erhalten, und Testen des DNA-Amplifikationsprodukts, um die Gegenwart oder Abwesenheit der WFFST1-Mutation zu bestimmen.

4. Verfahren nach Anspruch 3, wobei das Bestimmen der Gegenwart oder Abwesenheit der WFFST1-Mutation Folgendes umfasst: Abtrennen von RNA aus der biologischen Probe, Erzeugen einer cDNA aus der abgetrennten RNA und Testen der cDNA, um die Gegenwart oder Abwesenheit der WFFST1-Mutation zu bestimmen.

5. Verfahren nach Anspruch 1, wobei das Bestimmen der Gegenwart oder Abwesenheit der WFFST1-Mutation Folgendes umfasst: Isolieren von Protein aus der biologischen Probe und Testen des isolierten Proteins auf die Gegenwart eines Proteins, das die Sequenz von SEQ-ID-NR. 5 umfasst, wobei die Gegenwart eines Proteins, das die Sequenz von SEQ-ID-NR. 5 umfasst, die Gegenwart der WFFST1-Mutation feststellt.

6. Verfahren zur Bestimmung, ob ein Genom eines Pferdes eine Warmblood Fragile Foal Syndrome Typ 1 (WFFST1)-Mutation umfasst, wobei die WFFST1-Mutation eine Änderung von G zu A in der Position 4 in der Sequenz von SEQ-ID-NR. 6 umfasst, wobei das Verfahren Folgendes umfasst: Testen einer biologischen Probe von dem Pferd, um die Gegenwart oder Abwesenheit der WFFST1-Mutation zu bestimmen.

7. Verfahren nach Anspruch 6, wobei das Bestimmen der Gegenwart der Mutation das Bestimmen der Gegenwart von SEQ-ID-NR. 7 umfasst.

8. Verfahren nach Anspruch 6, wobei das Bestimmen der Abwesenheit der Mutation das Bestimmen der Gegenwart von SEQ-ID-NR. 6 umfasst.

9. Verfahren nach Anspruch 6, das Folgendes umfasst: Bestimmen der Abwesenheit der WFFST1-Mutation und Identifizieren des Pferdes, von dem die biologische Probe erhalten wurde, als nicht von WFFST1 betroffen.

10. Verfahren nach Anspruch 6, das Folgendes umfasst: Bestimmen der Gegenwart der WFFST1-Mutation und Identifizieren des Pferdes, von dem die biologische Probe erhalten wurde, als genetisch nicht normal bezüglich WFFST1.

11. Verfahren nach Anspruch 6, das Folgendes umfasst: Bestimmen der Homozygotie für die WFFST1-Mutation und Identifizieren des Pferdes, von dem die biologische Probe erhalten wurde, als von WFFST1 betroffen oder dafür prädisponiert.

12. Verfahren nach Anspruch 6, das Folgendes umfasst: Bestimmen der Heterozygotie für die WFFST1-Mutation und Identifizieren des Pferdes, von dem die biologische Probe erhalten wurde, als Träger der WFFST1-Mutation.

13. Verfahren nach Anspruch 6, wobei das Bestimmen der Gegenwart der WFFST1-Mutation das Nachweisen eines Polypeptids, das die Sequenz von SEQ-ID-NR. 5 umfasst, in der biologischen Probe umfasst.

14. Zusammensetzung, die eine isolierte Nukleinsäure umfasst, wobei die isolierte Nukleinsäure die Sequenz von SEQ-ID-NR. 1 umfasst.

## Revendications

1. Méthode de détermination du fait qu'un cheval soit hétérozygote, homozygote ou possède une absence d'une mutation du Syndrome du Poulain de Sang Fragile de Type 1 (WFFST1), où la mutation WFFST1 comprend un changement de G en A au niveau de la position 147 dans la séquence de SEQ ID n° 26, la méthode comprenant la détermination à partir d'un échantillon biologique issu du cheval de la présence ou de l'absence de la mutation WFFST1, et l'identification du cheval comme homozygote pour la mutation WFFST1 par la détermination de l'homozygosité pour la mutation WFFST1 ; ou l'identification du cheval comme hétérozygote pour la mutation WFFST1 par la détermination de l'hétérozygosité pour la mutation WFFST1 ; ou l'identification du cheval comme génétiquement normal en ce qui concerne le WFFST1 par la détermination d'une absence homozygote de la mutation WFFST1.

2. Méthode selon la revendication 1, dans laquelle la détermination de la présence ou de l'absence de la mutation WFFST1 comprend l'isolement d'ADN ou d'ARN à partir de l'échantillon biologique et l'analyse de l'ADN et/ou de l'ARN isolé pour la présence ou l'absence de la mutation WFFST1.

3. Méthode selon la revendication 1, dans laquelle la détermination de la présence ou de l'absence de la mutation WFFST1 comprend le traitement de l'échantillon biologique afin d'isoler l'ADN qui comprend ou peut comprendre la mutation WFFST1, puis l'amplification d'un segment de l'ADN isolé qui comprend ou peut comprendre la mutation WFFST1 afin d'obtenir un produit d'amplification d'ADN, et l'analyse du produit d'amplification d'ADN pour déterminer la présence ou l'absence de la mutation WFFST1.

4. Méthode selon la revendication 3, dans laquelle la détermination de la présence ou de l'absence de la mutation WFFST1 comprend la séparation d'ARN à partir de l'échantillon biologique, la création d'un ADNc à partir de l'ARN séparé, et l'analyse de l'ADNc pour déterminer la présence ou l'absence de la mutation WFFST1.

5. Méthode selon la revendication 1, dans laquelle la détermination de la présence ou de l'absence de la mutation WFFST1 comprend l'isolement de protéine à partir de l'échantillon biologique et l'analyse de la protéine isolée pour la présence d'une protéine comprenant la séquence de SEQ ID n° 5, où la présence d'une protéine comprenant la séquence de SEQ ID n° 5 établit la présence de la mutation WFFST1.

6. Méthode de détermination du fait que le génome d'un cheval comprenne une mutation du Syndrome du Poulain de Sang Fragile de Type 1 (WFFST1), où la mutation WFFST1 comprend un changement de G en A au niveau de la position 4 dans la séquence de SEQ ID n° 6, la méthode comprenant l'analyse d'un échantillon biologique issu du cheval pour déterminer la présence ou l'absence de la mutation WFFST1.

7. Méthode selon la revendication 6, dans laquelle la détermination de la présence de la mutation comprend la détermination de la présence de SEQ ID n° 7.

8. Méthode selon la revendication 6, dans laquelle la détermination de l'absence de la mutation comprend la détermination de la présence de SEQ ID n° 6.

9. Méthode selon la revendication 6, comprenant la détermination de l'absence de la mutation WFFST1 et l'identification du cheval à partir duquel l'échantillon biologique a été obtenu comme n'étant pas atteint du WFFST1.

10. Méthode selon la revendication 6, comprenant la détermination de la présence de la mutation WFFST1 et l'identification du cheval à partir duquel l'échantillon biologique a été obtenu comme n'étant pas génétiquement normal en ce qui concerne le WFFST1.

11. Méthode selon la revendication 6, comprenant la détermination de l'homozygosité pour la mutation WFFST1 et l'identification du cheval à partir duquel l'échantillon biologique a été obtenu comme étant atteint du ou prédisposé au WFFST1.

12. Méthode selon la revendication 6, comprenant la détermination de l'hétérozygosité pour la mutation WFFST1 et l'identification du cheval à partir duquel l'échantillon biologique a été obtenu comme étant porteur de la mutation WFFST1.

13. Méthode selon la revendication 6, dans laquelle la détermination de la présence de la mutation WFFST1 comprend la détection à partir de l'échantillon biologique d'un polypeptide comprenant la séquence de SEQ ID n° 5.

14. Composition comprenant un acide nucléique isolé, dans laquelle l'acide nucléique isolé comprend la séquence de SEQ ID n° 1.
